# FASCICULE DE BREVET EUROPEEN

(11) **EP 0 854 879 B1**
(45) Date de publication et mention de la délivrance du brevet: **16.01.2002**
(21) Numéro de dépôt: 96933501.7
(22) Date de dépôt: 08.10.1996
(51) Int. Cl.: C07H 17/00, A61K 31/70

(54) **NOUVEAUX DERIVES DE LA 5-O-DESOSAMINYL 6-O-METHYL ERYTHRONOLIDE A, LEUR PROCEDE DE PREPARATION ET LEUR APPLICATION A LA PREPARATION DE PRODUITS BIOLOGIQUEMENT ACTIFS**
DERIVATE VON 5-0-DESOSAMINYL-6-0-METHYL ERYTHRONOLID A, IHRE VERFAHREN ZUR HERSTELLUNG UND VERWENDUNG ZUR HERSTELLUNG VON ARZNEIMITTELN
NOVEL 5-O-DEOSAMINYL 6-O-METHYL ERYTHRONOLIDE A DERIVATIVES, PREPARATION METHOD THEREFOR AND USE THEREOF FOR PREPARING BIOLOGICALLY ACTIVE MATERIALS

(30) Priorité: 09.10.1995 FR 9511861
(43) Date de publication de la demande: 29.07.1998
(73) Titulaire: Aventis Pharma S.A., 92160 Antony (FR)
(72) Inventeur: BONNET, Alain, F-77100 Meaux (FR); CHAPPERT, Bernadette, F-75020 Paris (FR); LAGOUARDAT, Jacques, F-93160 Noisy-le-Grand (FR)
(86) Numéro de dépôt international: FR9601567
(87) Numéro de publication internationale: WO9713774

(56) Documents cités:
- EP-A- 0 619 320
- EP-A- 0 638 585
- EP-A- 0 682 038
- FR-A- 2 692 579

## Description

La présente invention concerne de nouveaux dérivés de la 5-0-désosaminyl 6-O-méthyl érythronolide A, leur procédé de préparation et leur application à la préparation de produits biologiquement actifs.

L'invention a pour objet les composés de formule (I) : 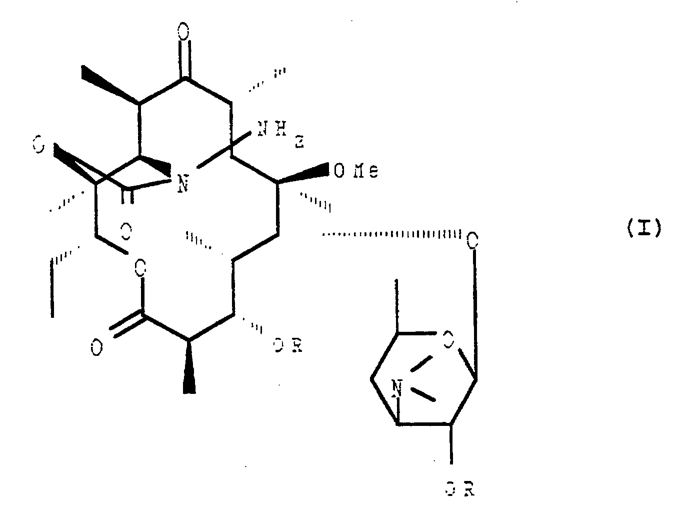 dans lesquels R représente le reste d'un acide carboxylique renfermant jusqu'à 18 atomes de carbone.

Parmi les restes d'acide carboxylique, on peut citer notamment les radicaux acétyle, propionyle, butyryle, isobutyryle, n-valéryle, isovaléryle, tert-valéryle et pivalyle.

L'invention a plus particulièrement pour objet les composés de formule (I) dans lequel R représente un radical acétyle.

L'invention a également pour objet un procédé de préparation caractérisé en ce que l'on soumet un composé de formule (II) : 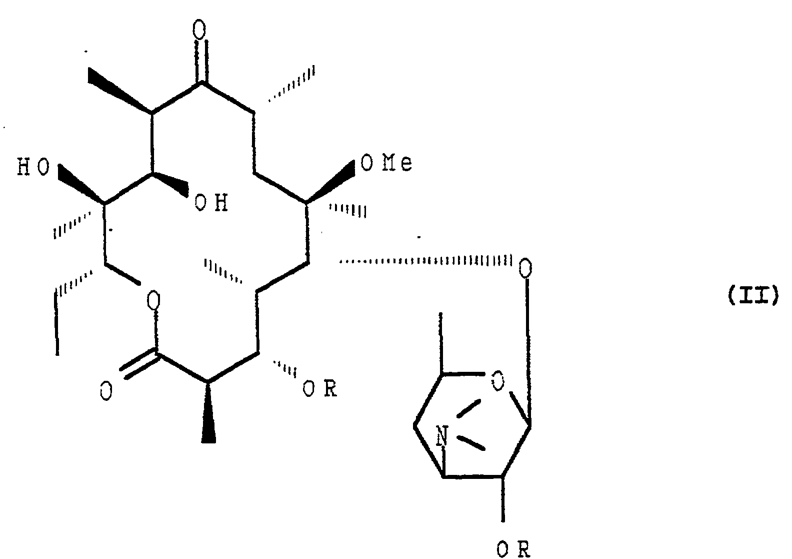 dans lequel R conserve sa signification précédente à l'action d'un agent capable d'activer sélectivement l'hydroxyle en 11 choisi dans le groupe constitué par un dérivé d'acide sulfonique et le chlorure de thionyle, puis à l'action d'une base pour obtenir le composé de formule (III) : 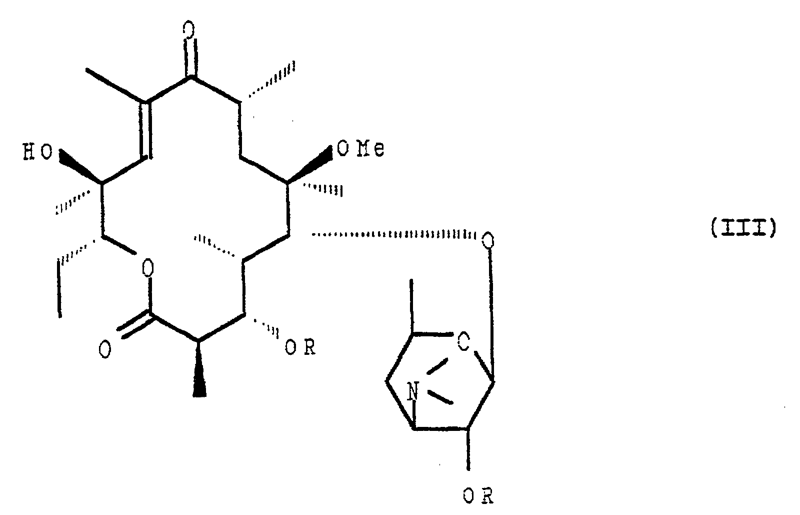 que l'on soumet à l'action du carbonyidiimidazole, puis à l'action de l'hydrazine NH₂NH₂ pour obtenir le composé de formule (I) correspondant.

Les composés de formule (II) sont connus d'une façon générale et peuvent être préparés selon le procédé décrit dans la demande de brevet européen 619319.

Dans un mode de réalisation préféré,
- l'agent capable d'activer sélectivement l'hydroxyle en 11 est un dérivé d'acide sulfonique tel que l'anhydride méthanesulfonique, paratoluènesulfonique, trifluorométhanesulfonique ou le chlorure de thionyle SOCl₂, lequel forme un sulfite cyclique avec la fonction OH en 12,
- la base utilisée pour créer une double liaison 10(11) est un diazabicycloundécène, par exemple le DBU (ou 1,8-diazabicyclo[5-4-0]undec-7-ène), ou le DBN (ou 1,5-diazabicyclo[4,3,0]non-5-ène ou la 2,6-lutidine, ou la 2,4,6-collidine ou la tétraméthylguanidine,
- la réaction du composé de formule (III) avec le carbonyldiimidazole a lieu en présence d'une des bases citées ci-dessus ou encore en présence d'hydrure de sodium, de triéthylamine, de carbonate ou de carbonate acide de sodium ou de potassium, NaN(SiMe₃)₂ ou LiN(SiMe₃)₂,
- l'hydrazine est utilisée sous forme d'hydrate d'hydrazine.

Les composés de formule (III) obtenus sont des produits nouveaux et sont en eux-mêmes un objet de la présente invention.

L'invention a donc pour objet à titre de produits chimiques les composés de formule (III) et tout particulièrement le composé de formule (III) dans lequel R représente un radical acétyle.

La demande de brevet FR 2 692 579 décrit des dérivés de la picromycine, présentant d'intéressantes propriétés antibiotiques, et leur procédé de préparation impliquant l'action directe d'un composé 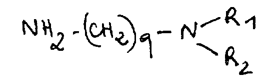 sur le composé issu de l'action du carbonyl diimidazole sur le composé de formule (III) (=dérivé de la picromycine analogue au composé de formule (III) de l'invention) que l'on soumet à l'action d'un agent d'estérification pour obtenir le composé de formule (VI') correspondant à la formule VI dans laquelle l'hydroxyle en 2' est remplacé par OR.

Les composés de formule (I) sont des produits intermédiaires intéressants, qui peuvent conduire notamment à la préparation de produits antibiotiques, décrits et revendiqués dans la demande de brevet européen 0 676 409.

L'invention a notamment pour objet l'application, caractérisée en ce que l'on soumet un composé de formule (I) à l'action d'un agent de clivage des fonctions hydroxyles protégées pour obtenir le composé de formule (IV) : 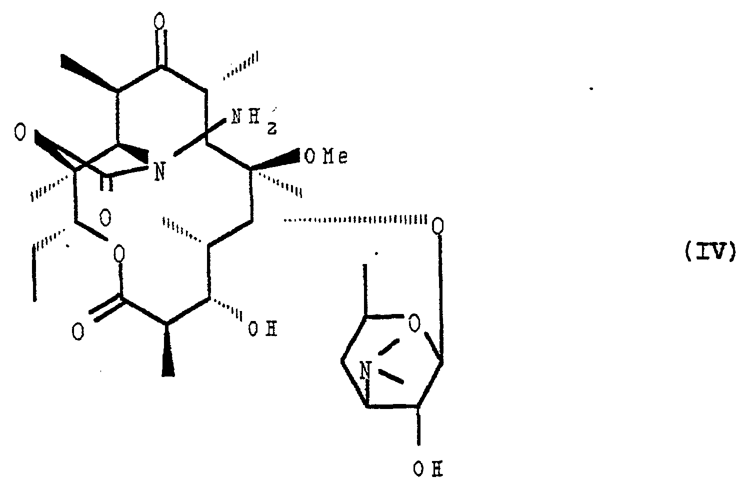 que l'on soumet à l'action d'un aldéhyde de formule (V) : 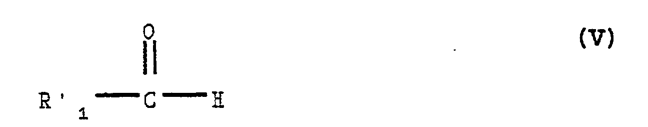 dans lequel R'₁ représente un atome d'hydrogène ou un radical hydrocarboné renfermant jusqu'à 23 atomes de carbone, saturé ou insaturé, éventuellement interrompu par un ou plusieurs hétéroatomes et portant éventuellement un ou plusieurs groupements fonctionnels pour obtenir le composé de formule (VI) : 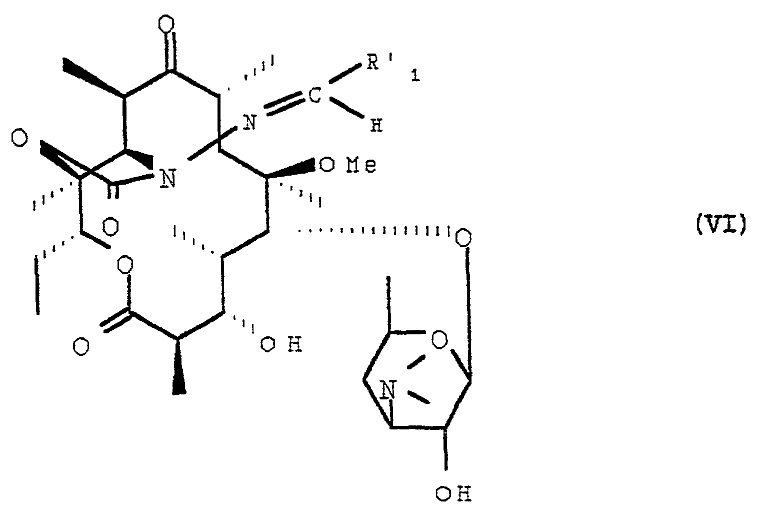 que l'on soumet à l'action d'un agent d'oxydation de l'hydroxyle en 3 puis à l'action d'un agent de clivage de l'hydroxyle en 2' pour obtenir le composé de formule (VII) : 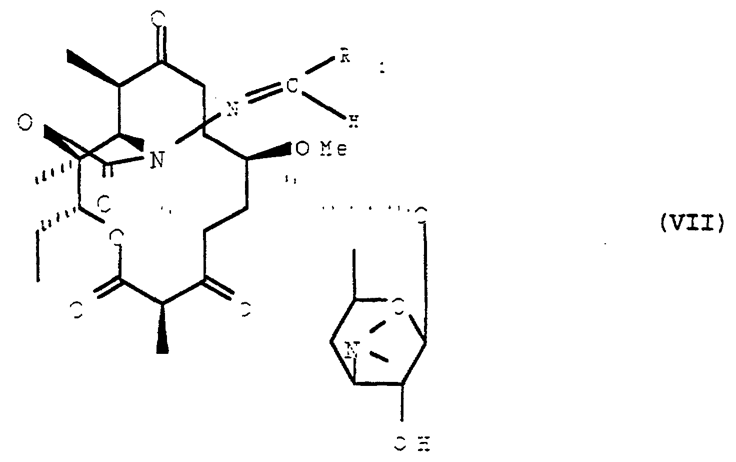 que l'on soumet à l'action d'un agent de réduction pour obtenir le composé de formule (VIII) correspondant : 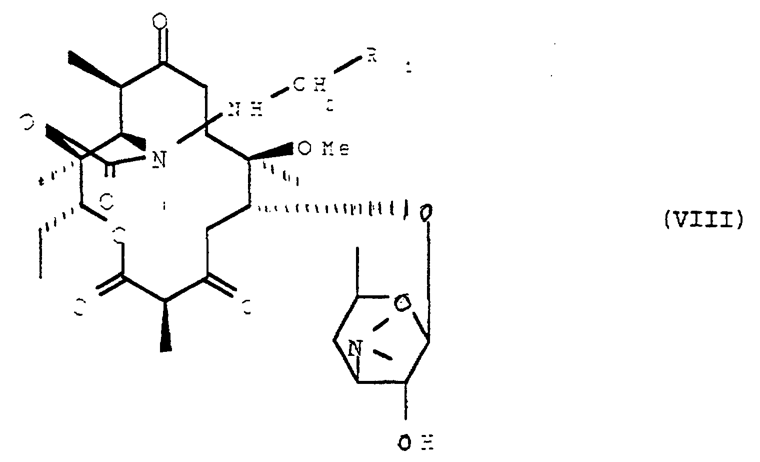 dans laquelle R'₁ conserve sa signification précédente.

Dans un mode de réalisation préféré,
- R'₁ représente le radical 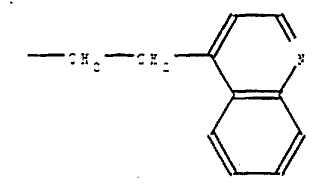
- le clivage des fonctions hydroxyles protégées est réalisé par saponification puis acidification de la fonction ester,
- l'estérification de la fonction hydroxyle en 2' est réalisée selon les procédés classiques,
- l'oxydation de l'hydroxyle en 3 est effectuée en utilisant un diimide en présence de DMSO, par exemple le chlorhydrate de 1-éthyl 3-(3-diméthylamino propyl) carbodiimide,
- le clivage de l'hydroxyle en 2' a lieu par méthanolyse,
- l'agent de réduction est NaBH₃CN ou NaBH(OAc)₃ ou encore NaBH₄ en présence d'acide acétique ou l'hydrogène en présence d'un catalyseur tel que le palladium, le platine et éventuellement en présence d'un acide tel que l'acide chlorhydrique ou l'acide acétique.

Les produits de formule (VII) sont des produits présentant des propriétés antibiotiques intéressantes, décrits et revendiqués dans le brevet européen 0676409.

### EXEMPLE 1 : 2',3-diacétate de 11,12-didéoxy-3-de(2,6-didéoxy-3-C-méthyl-3-O-méthyl-alpha-L-ribohexopyranosyl) -11,12-(hydrazono(carbonyloxy)-6-O-méthyl-érythromycine

### STADE A : 2',3-diacétate de 11-déoxy-10,11-didéhydro-3-de(2,6-didéoxy-3-C-méthyl-3-O-méthyl-alpha-L-ribohexopyranosyl)-6-0-méthyl-érythromycine

On maintient sous agitation pendant 15 minutes à 0°C une solution renfermant 9,45 g de 2',3-diacétate de 3-de(2,6-didéoxy-3-C-méthyl-3-O-méthyl-alpha-L-ribohexopyranosyl)-6-O-méthyl-érythromycine et 112 ml de pyridine. On ajoute en 10 minutes, 1,52 ml de chlorure de thionyle. On maintient l'agitation pendant une nuit à la température ambiante. On essore et sèche. On verse dans un mélange de 150 ml d'acétate d'éthyle et 200 ml de bicarbonate de soude. On agite pendant 10 minutes. On décante, extrait à l'acétate d'éthyle et sèche. On obtient 10,7 g de produit.

On agite à 50°C un mélange de 10,7 g de ce produit-et 124 ml de diméthylformamide. On ajoute en 5 minutes 2,53 ml de DBU. On agite 48 heures à 50°C, verse dans l'eau. On ajoute 100 cm³ d'acétate d'éthyle. On décante, lave à l'eau (1,25 1), extrait à l'acétate d'éthyle (400 ml) et sèche sur sulfate de magnésium, filtre et évapore à sec le filtrat. On ajoute environ 50 ml d'éther isopropylique, laisse la cristallisation se faire pendant 72 heures, filtre, rince et sèche. On obtient 5,514 g de produit recherché fondant à 174°C.

### STADE B : 2',3-diacétate de 11,12-didéoxy-3-de(2,6-didéoxy-3-C-méthyl-3-O-méthyl-alpha-L-ribohexopyranosyl)-11,12-(hydrazono(carbonyloxy)-6-O-méthyl-érythromycine

On agite un mélange renfermant 2,623 g de produit préparé au stade précédent, 972 mg de carbonyldiimidazole, 30 ml de dichlorométhane et 60 µl de DBU. On maintient l'agitation pendant 4 heures. On ajoute 404 µl d'hydrate d'hydrazine. On agite pendant 24 heures, ajoute 50 ml de phosphate acide de sodium 0,5 M. On décante, extrait au chlorure de méthylène, sèche. On reprend dans l'éther isopropylique. On laisse cristalliser pendant une nuit. On filtre, rince à l'éther isopropylique et sèche. On obtient 2,415 g de produit recherché.

| RMN CDCl₃ ppm | |
|---|---|
| 0,84 (t) | CH₃-CH₂ |
| 1,00 (d) 1,10 (d) | |
| 1,12 (d)-1,15 (d) | les CH-Me |
| 1,23 (d) | |
| 1,28 (s) | 6 et 12 Me |
| 1,40 (S) | |
| 2,09 (s) | les OAc |
| 2,18 (s) | |
| 2,26 (s) | NMe₂ |
| 2,61 (m) (2H) | H₈ et H'₃ |
| 2,88 (m) | H₂ |
| ≅ 3,06 (m) | H₁₀ |
| 3,02 (s) | C-OMe |
| 3,33 (m) | H'₅ |
| 3,67 (s) | H₁₁ |
| 3,69 (d) | H₅ |
| 4,03 (d) | H'₁ax |
| 4,53 (s1) (2H) | NH₂ |
| 4,73 (dd) | H'₂ax |
| 5,03 (d) | H₃ |
| 5,13 (dd) | H₁₃ |

### APPLICATION : 11,12-didéoxy-3-de((2,6-didéoxy-3-C-méthyl-3-O-méthyl-alpha-L-ribohexopyranosyl) oxy) 6-O-méthyl 3-oxo 12,11-(oxycarbonyl) (2-(3-(4-quinoléinyl) 2-propyl) hydrazona)) erythromycine

### STADE A : 11,12-didéoxy-3-de(2,6-didéoxy-3-C-méthyl-3-O-méthyl-alpha-L-ribohexopyranosyl) 11,12-(hydrazono (carbonyloxy) 6-O-méthyl érythromycine

On agite pendant 30 minutes un mélange renfermant 714 mg de produit de l'exemple 1 stade B, 7,5 ml d'isopropanol et 2 ml de soude N. On maintient le mélange réactionnel pendant 48 heures à la température ambiante. On ajoute 2 ml d'une solution normale d'acide chlorhydrique. On évapore à sec. On chromatographie le produit obtenu sur silice en éluant avec le mélange acétate d'éthyle-triéthylamine (9-1). On obtient 300 mg de produit recherché.

### STADE B : 2'-acétate de 11,12-didéoxy-3-de(2,6-didéoxy-3-C-méthyl-3-O-méthyl-alpha-L-ribohexopyranosyl) 12,11-(oxycarbonyl) (3-(4-quinoléinyl) propylidène) hydrazono) 6-O-méthyl érythromycine

On maintient sous agitation et atmosphère d'azote pendant 4 heures, 281 ml de 4-quinoléinylpropanaldéhyde, 10,2 ml de toluène, 802 mg de produit préparé au stade précédent et 306 µl d'acide acétique. On évapore à sec. On chromatographie le produit obtenu sur silice en éluant avec le mélange acétate d'éthyle triéthylamine (95-5), puis avec le mélange acétate d'éthyle-triéthylamine (90-10). On obtient 916 mg du produit.

On maintient sous agitation pendant une nuit 839 mg de ce produit, 10 ml de chlorure de méthylène et 121 µl d'anhydride acétique. On ajoute 8,55 ml d'eau ammoniaquée. On agite pendant 10 minutes, extrait au chlorure de méthylène et sèche. On obtient 846 mg de produit recherché.

### STADE C : 11,12-didéoxy-3-de((2,6-didéoxy-3-C-méthyl-3-O-méthyl-alpha-L-ribohexopyranosyloxy) 3-oxo 12,11-(oxycarbonyl (3-(4-quinoléinyl) propylidène) hydrazono)) 6-0-méthyl érythromycine

On agite pendant 15 minutes, un mélange renfermant 1,783 g de chlorhydrate 1-éthyl 3-(3-diméthylamino propylcarbodiimide), 1,67 ml de DMSO et 11 ml de chlorure de méthylène. On ajoute 781 mg de produit préparé au stade précédent et 8 ml de chlorure de méthylène. On maintient sous agitation pendant 1,30 heures et ajoute 1,8 g de trifluoroacétate de pyridinium. On agite pendant 3 heures à la température ambiante, et ajoute 30 ml d'ammoniaque. On maintient sous agitation pendant 15 minutes, extrait au chlorure de méthylène et sèche sur sulfate de magnésium. On chromatographie sur silice en éluant avec le mélange acétate d'éthyle-triéthylamine (9-1). On recueille 647 mg de produit. On maintient pendant une nuit sous agitation, un mélange de 566 mg de ce produit et 18 ml de méthanol et obtient 540 mg de produit recherché.

### STADE D : 11,12-didéoxy 3-de((2,6-didéoxy 3-C-méthyl 3-O-méthyl alpha-L-ribohexopyranosyl) oxy) 6-O-méthyl 3-oxo 12,11(oxycarbonyl (2-(3-(4-quinoléinyl) 2-propyl) hydrazono)) érythromycine

On met en solution dans 10 ml d'acétate d'éthyle, 0,38 g de produit préparé au stade précédent et 38 mg d'oxyde de platine. On hydrogène sous vive agitation pendant 24 heures. On filtre, rince à l'acétate d'éthyle et évapore sous pression réduite. On obtient 0,375 g de produit que l'on reprend dans 5 ml de méthanol, 175 µl d'acide acétique et 90 mg de borohydrure de sodium. On agite 3 heures à la température ambiante. On chasse le méthanol et reprend à l'aide du mélange chlorure de méthylène-eau. On amène à pH 8-9 avec une solution d'ammoniaque à 28 %. On décante, lave à l'eau, sèche, filtre et évapore à sec. On obtient 0,37 g de produit que l'on chromatographie sur silice, en éluant avec un mélange acétate d'éthyle-triéthylamine 96-4. On obtient 127 mg d'un produit (rf = 0,25) que l'on essore, lave et sèche. On obtient 90 mg du produit recherché F = 189°C. RMN CDCl₃ ppm, 300 MHz 1,34 (s)-1,48 (s) : 6 et 12 CH₃ ; 2,30 (s) : N(CH₃)₂ ; 2,65 (s) : 6-OCH₃ ; 3,06 (dq) : H₄ ; 3,19 (q) : H₁₀ ; 3,74 (s) : H₁₁ ; 5,50 (t. mobile) : NH-CH₂ ; 7,30 (d) : H₃ quinoléine ; 7,53-7,68 (dt) : H₆-H₇ quinoléine ; 8,10 (m) : H₅-H₈ quinoléine ; 8,79 (d) : H₂ quinoléine.

## Revendications

1. Les composés de formule (I) : 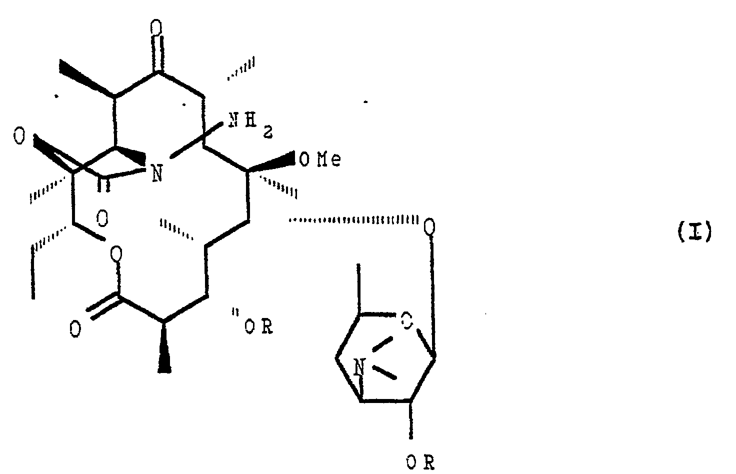 dans lesquels R représente le reste d'un acide carboxylique renfermant jusqu'à 18 atomes de carbone.

2. Le composé de formule (I) selon la revendication 1, dans lequel R représente un radical acétyle.

3. Procédé de préparation des composés de formule (I) tels que définis à la revendication 1 ou 2, **caractérisé en ce que** l'on soumet un composé de formule (II) : 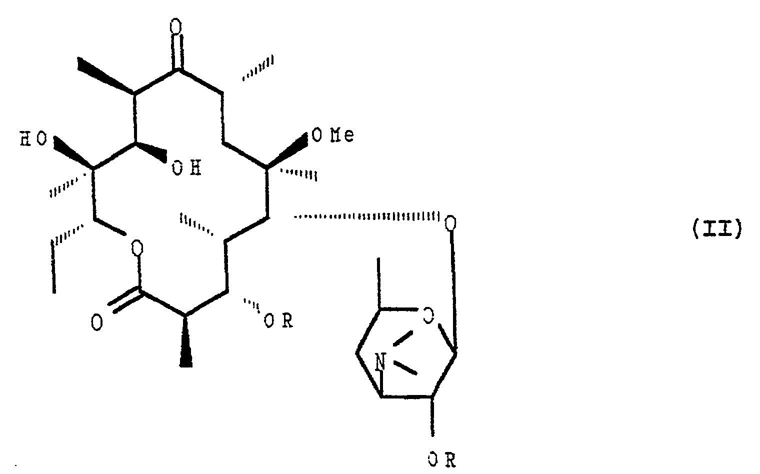 dans lequel R conserve sa signification précédente à l'action d'un agent capable d'activer sélectivement l'hydroxyle en 11, choisi dans le groupe constitué par un dérivé d'acide sulfonique et le chlorure de thionyle, puis à l'action d'une base pour obtenir le composé de formule (III) : 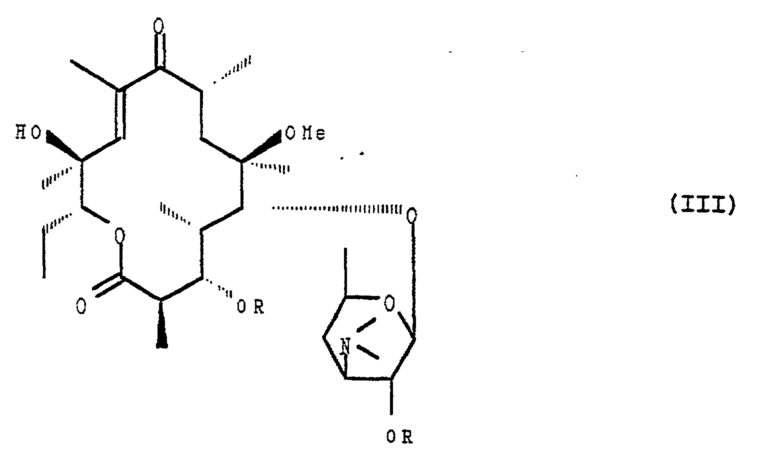 que l'on soumet à l'action du carbonyldiimidazole, puis à l'action de l'hydrazine NH₂NH₂ pour obtenir le composé de formule (I) correspondant.

4. A titre de produits chimiques, les composés de formule (III) selon la revendication 3.

5. A titre de produit chimique, le composé de formule (III) dans lequel R représente un radical acétyle.

6. Application des composés de formule (I) tels que définis à la revendication 1 ou 2, **caractérisée en ce que** l'on soumet un composé de formule (I) à l'action d'un agent de clivage des fonctions hydroxyles protégées pour obtenir le composé de formule (IV) : 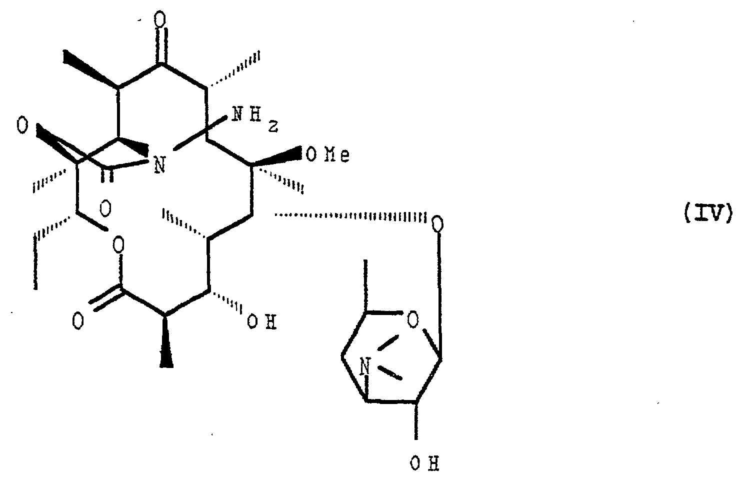 que l'on soumet à l'action d'un aldéhyde de formule (V) : 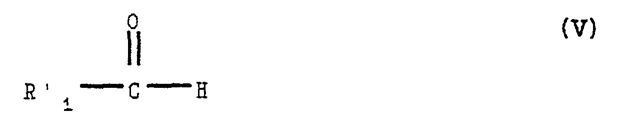 dans lequel R'₁ représente un atome d'hydrogène ou un radical hydrocarboné renfermant jusqu'à 23 atomes de carbone, saturé
ou insaturé, éventuellement interrompu par un ou plusieurs hétéroatomes et portant éventuellement un ou plusieurs groupements fonctionnels pour obtenir le composé de formule (VI) : 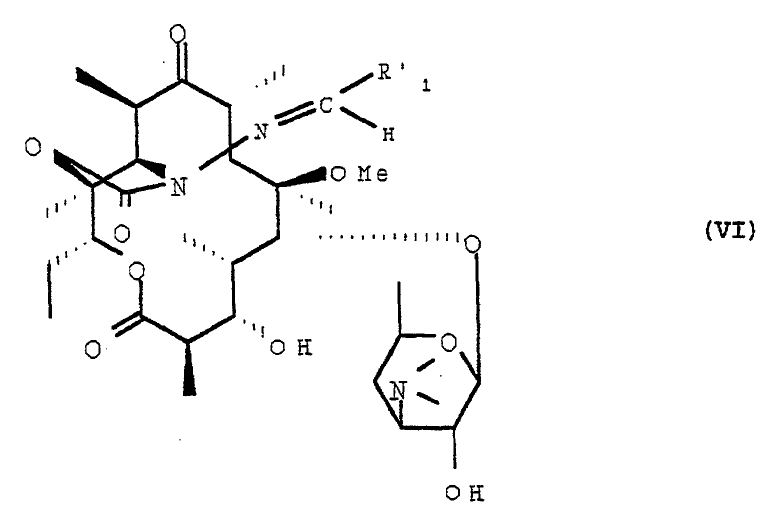 que l'on soumet à l'action d'un agent d'estérification pour obtenir le composé de formule (VI') correspondant à la formule VI dans laquelle l'hydroxyle en 2' est remplacé par OR,
que l'on soumet à l'action d'un agent d'oxydation de l'hydroxyle en 3 puis à l'action d'un agent de clivage de l'hydroxyle en 2' pour obtenir le composé de formule (VII) : 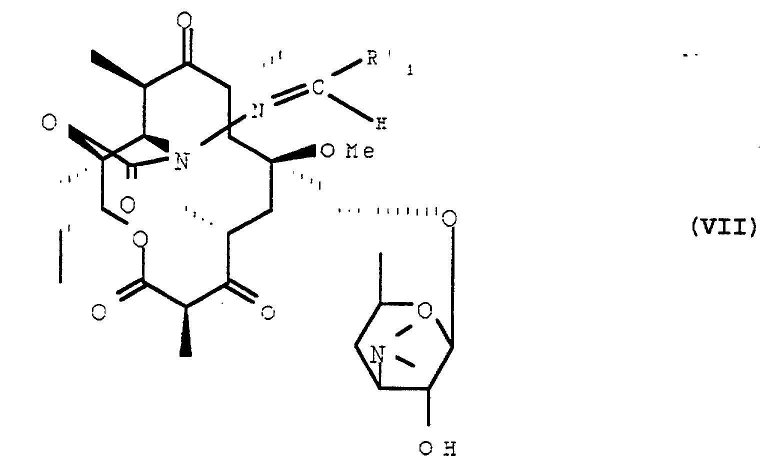 que l'on soumet à l'action d'un agent de réduction pour obtenir le composé de formule (VIII) correspondant : 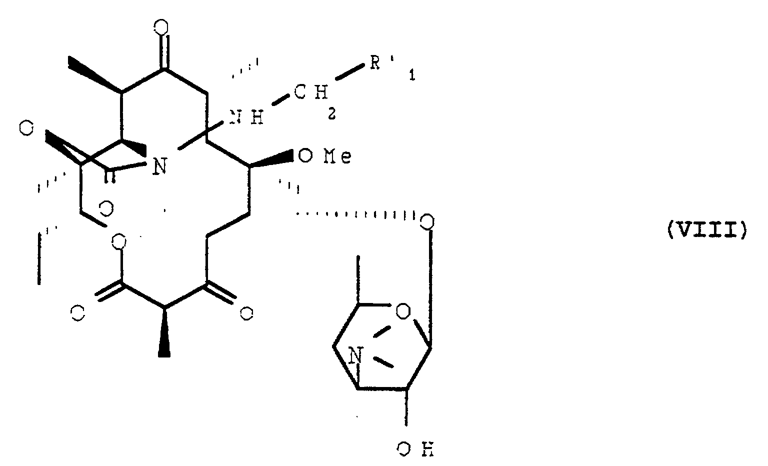 dans laquelle R'₁ conserve sa signification précédente.

7. Application des composés de formule (I) à la préparation du composé de formule (VIII) dans lequel R'₁ représente un radical

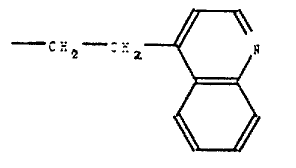

## Claims

1. The compounds of formula (I): 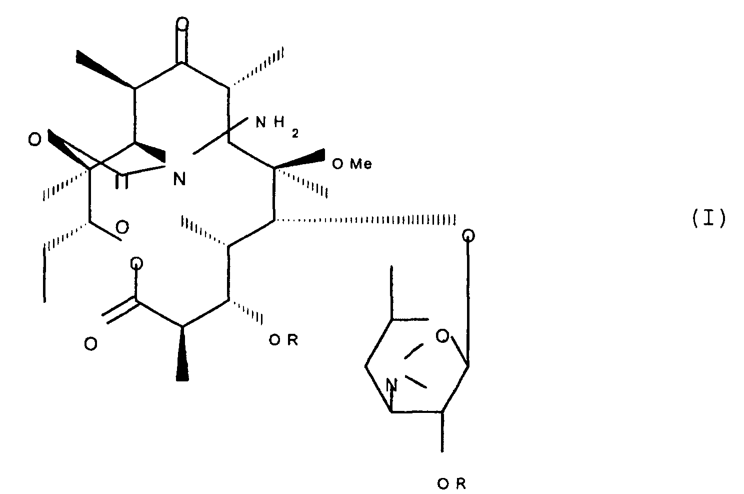 in which R represents the remainder of a carboxylic acid containing up to 18 carbon atoms.

2. The compound of formula (I) according to claim 1, in which R represents an acetyl radical.

3. Process for the preparation of the compounds of formula (I) as defined in claim 1 or 2, **characterized in that** a compound of formula (II): 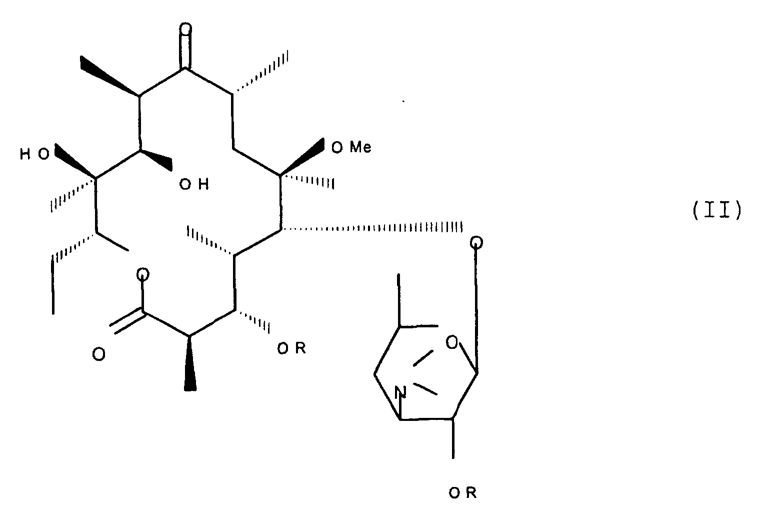 in which R retains its previous meaning is subjected to the action of an agent capable of selectively activating the hydroxyl in position 11 chosen from the group constituted by a sulphonic acid derivative and thionyl chloride, then to the action of a base in order to obtain the compound of formula (III): 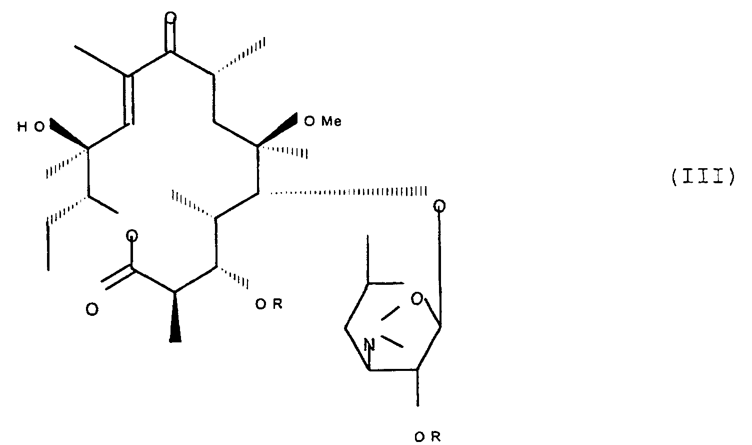 which is subjected to the action of carbonyldiimidazole, then to the action of hydrazine NH₂NH₂ in order to obtain the corresponding compound of formula (I).

4. As chemical products, the compounds of formula (III) according to claim 3.

5. As a chemical product, the compound of formula (III) in which R represents an acetyl radical.

6. Use of the compounds of formula (I) as defined in claim 1 or 2, **characterized in that** a compound of formula (I) is subjected to the action of a cleavage agent of the protected hydroxyl functions in order to obtain the compound of formula (IV) : 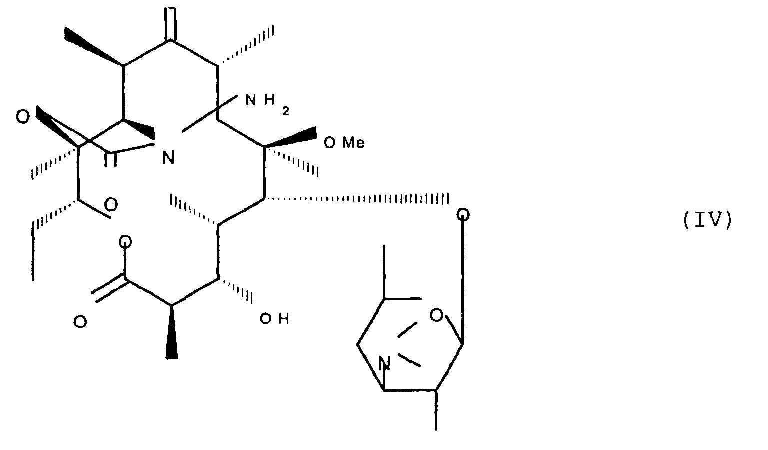 which is subjected to the action of an aldehyde of formula (V) : 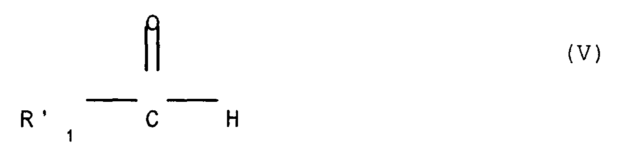 in which R'₁ represents a hydrogen atom or a hydrocarbon radical containing up to 23 carbon atoms, saturated or unsaturated, optionally interrupted by one or more heteroatoms and optionally carrying one or more functional groups in order to obtain the compound of formula (VI): 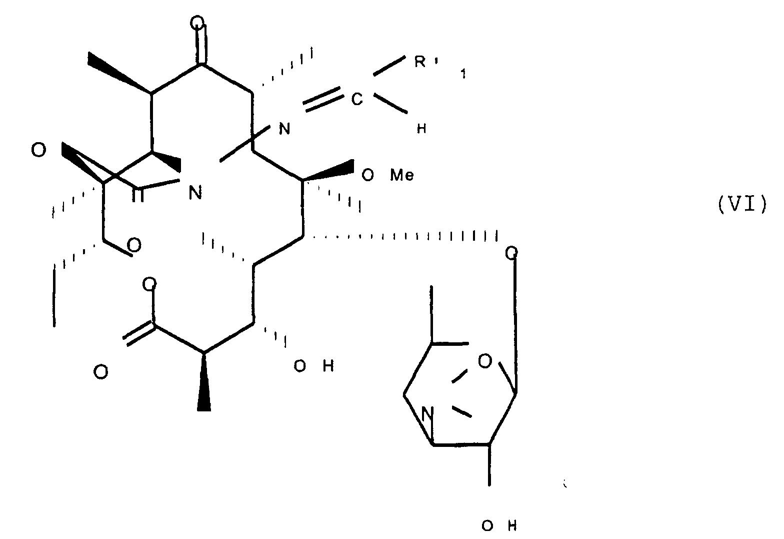
which is subjected to the action of an esterification agent in order to obtain the compound of formula (VI') corresponding to formula VI in which the hydroxyl in position 2' is replaced by OR,
which is subjected to the action of an oxidation agent of the hydroxyl in position 3 then to the action of a cleavage agent of the hydroxyl in position 2' in order to obtain the compound of formula (VII): 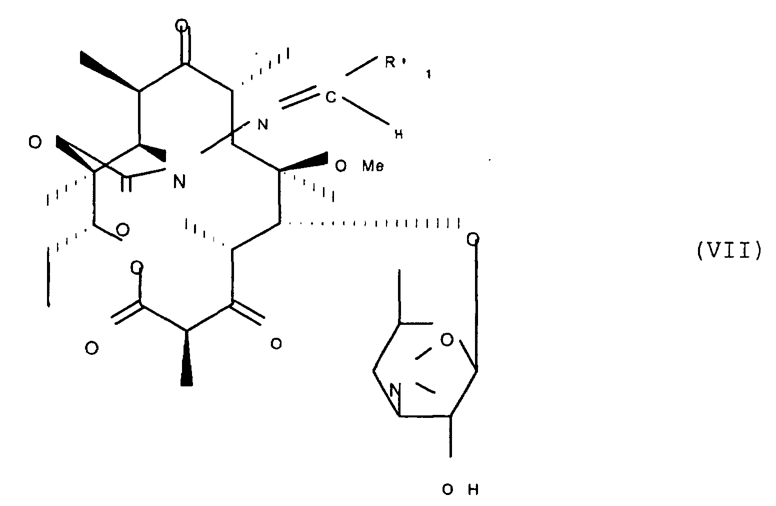
which is subjected to the action of a reducting agent in order to obtain the corresponding compound of formula (VIII): 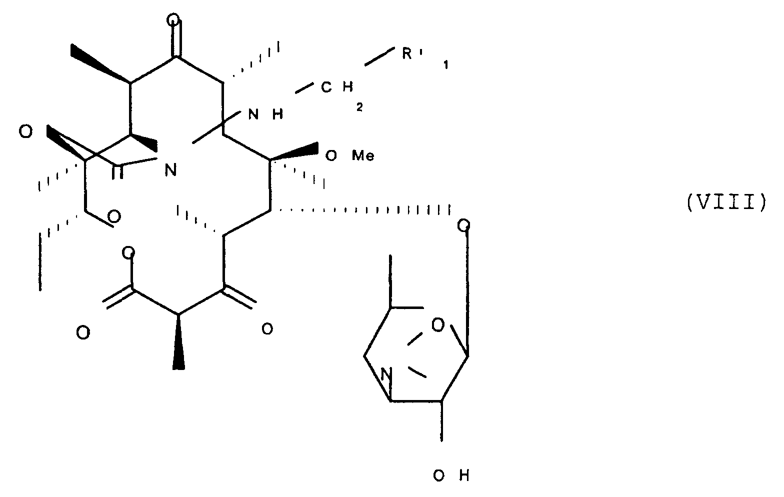
in which R'₁ retains its previous meaning.

7. Use of the compounds of formula (I) for the preparation of the compound of formula (VIII) in which R'₁ represents a 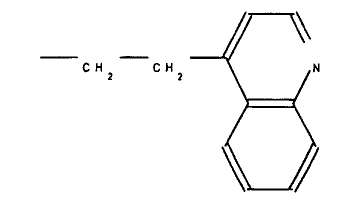 radical

## Patentansprüche

1. Die Verbindungen der Formel (I): 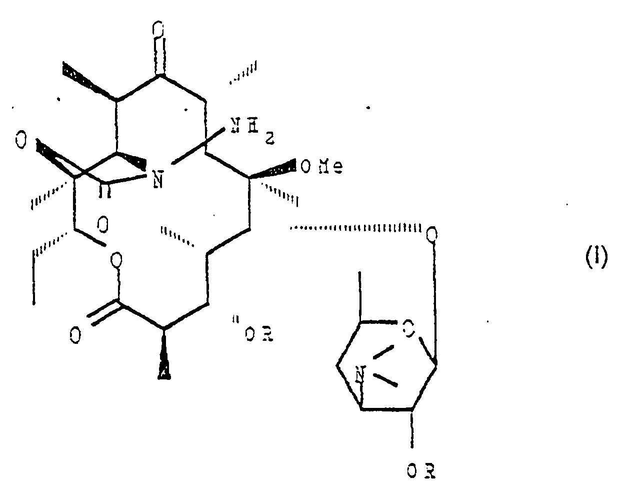 in denen R den Rest einer Carbonsäure mit bis zu 18 Kohlenstoffatomen darstellt.

2. Die Verbindung der Formel (I) gemäß Anspruch 1, in der R einen Acetylrest darstellt.

3. Verfahren zur Herstellung der Verbindungen der Formel (I), wie in Anspruch 1 oder 2 definiert, **dadurch gekennzeichnet, dass** man eine Verbindung der Formel (II): 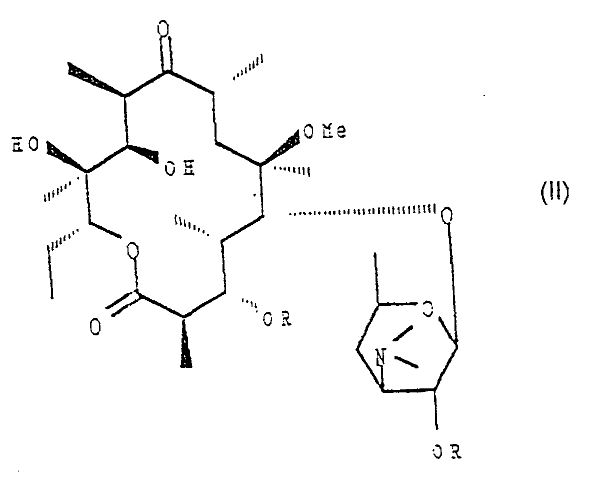 in der R seine vorherige Bedeutung behält, der Einwirkung eines Mittels, das die Hydröxygruppe in Position 11 selektiv aktivieren kann, das aus der Gruppe ausgewählt ist, die aus einem Sulfonsäurederivat und Thionylchlorid besteht, dann der Einwirkung einer Base unterzieht, um die Verbindung der Formel (III): 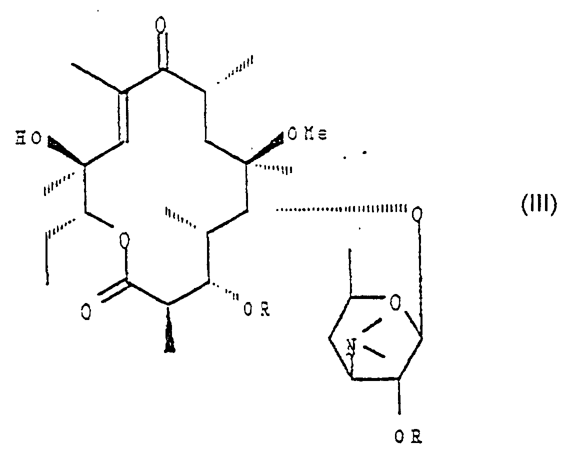 zu erhalten, die man der Einwirkung von Carbonyldiimidazol, dann der Einwirkung von Hydrazin NH₂NH₂ unterzieht, um die entsprechende Verbindung der Formel (I) zu erhalten.

4. Als chemische Produkte die Verbindungen der Formel (III) gemäß Anspruch 3.

5. Als chemisches Produkt die Verbindung der Formel (III), in der R einen Acetylrest darstellt.

6. Verwendung der Verbindungen der Formel (I), wie in Anspruch 1 oder 2 definiert, **dadurch gekennzeichnet, dass** man eine Verbindung der Formel (I) der Einwirkung eines Mittels zur Spaltung der geschützten Hydroxyfunktionen unterzieht, um die Verbindung der Formel (IV): 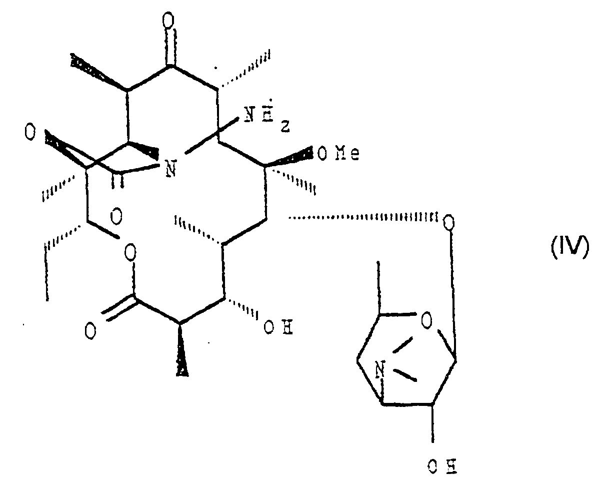 zu erhalten, die man der Einwirkung eines Aldehyds der Formel (V): 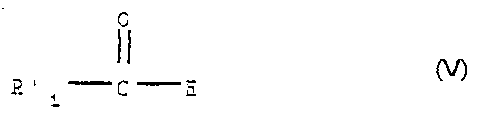 unterzieht, in der R'₁ ein Wasserstoffatom oder einen gesättigten oder ungesättigten Kohlenwasserstoffrest mit bis zu 23 Kohlenstoffatomen darstellt, der gegebenenfalls von einem oder mehreren Heteroatomen unterbrochen ist und der gegebenenfalls eine oder mehrere funktionelle Gruppen trägt, um die Verbindung der Formel (VI): 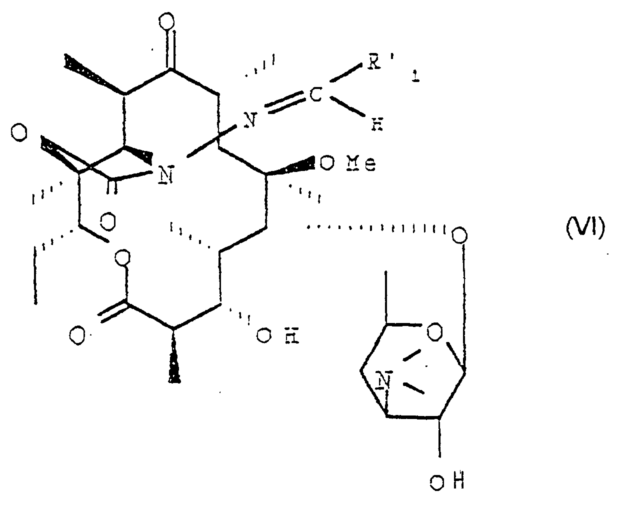 zu erhalten, die man der Einwirkung eines Veresterungsmittels unterzieht, um die Verbindung der Formel (VI') zu erhalten, die der Formel VI, in der die Hydroxygruppe in Position 2' durch OR ersetzt ist, entspricht, die man der Einwirkung eines Mittels zur Oxidation der Hydroxygruppe in Position 3, dann der Einwirkung eines Mittels zur Spaltung der Hydroxygruppe in Position 2' unterzieht, um die Verbindung der Formel (VII): 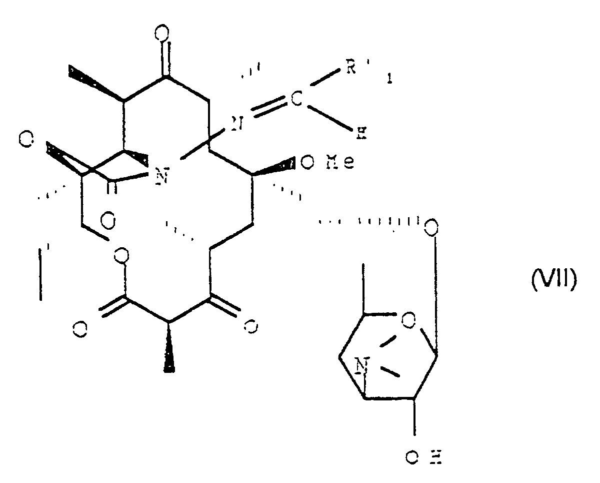 zu erhalten, die man der Einwirkung eines Reduktionsmittels unterzieht, um die entsprechende Verbindung der Formel (VIII): 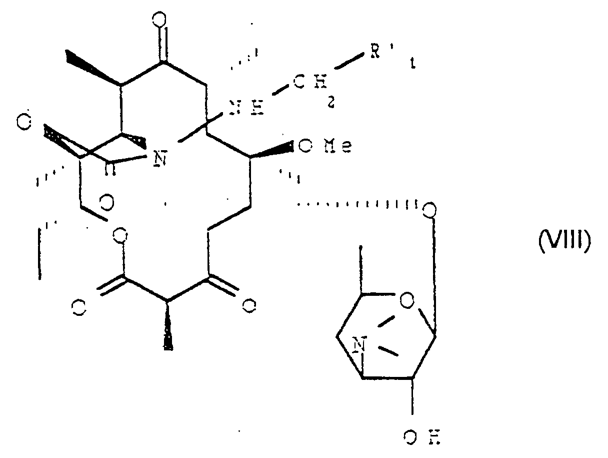 in der R'₁ seine vorherige Bedeutung behält, zu erhalten.

7. Verwendung der Verbindungen der Formel (I) bei der Herstellung der Verbindung der Formel (VIII), in der R'₁ einen Rest 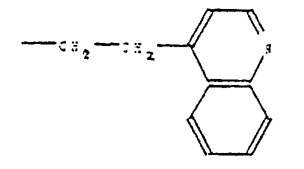 darstellt.
